# EUROPEAN PATENT APPLICATION

(11) **EP 4 059 566 A1**
(43) Date of publication of application: **21.09.2022**
(21) Application number: 22161801.0
(22) Date of filing: 14.03.2022
(51) Int. Cl.: A61N 2/00

(54) **SYSTEM FOR FACILITATING DELIVERY OF TRANSCRANIAL MAGNETIC STIMULATION**

(30) Priority: 15.03.2021 FI 20215275
(71) Applicant: Nexstim Oyj, 00510 Helsinki (FI)
(72) Inventor: JÄRNEFELT, Gustaf, 00510 Helsinki (FI)
(74) Representative: Laine IP Oy

(57) **Abstract**

An apparatus and method for facilitating delivery of Transcranial Magnetic Stimulation (TMS) by: receiving anatomical data for a person (170) defining a shape of at least the cranium and an outer surface of the skin surrounding the cranium; receiving information from a tracking system (130) regarding a real-time position and orientation of the head of the person (170); and controlling a multi-axis robotic arm (120) to maintain a TMS stimulation device (110) at an optimal position and orientation relative to the head based on the anatomical data and the information regarding the real-time position and orientation of the head in order to target a predetermined location within the cranium with the TMS stimulation device (110); wherein the optimal position and orientation is such that a surface of the TMS stimulation device (110) is a predetermined distance from the outer surface of the skin.

## Description

### FIELD

The present invention relates to Transcranial Magnetic Stimulation (TMS) and TMS enabled via a robotic arm.

### BACKGROUND

When delivering TMS, accurate and repeatable positioning of TMS stimulation devices, such as coils, is critical. While many solutions exist for holding and positioning TMS stimulation devices, such solutions rely on placing the TMS stimulation device firmly against the head in an attempt to maximize stimulation delivery and minimize the movement of the head. However, pressing the TMS stimulation device against the head can cause discomfort and even result in a person retreating from the TMS stimulation device due to this discomfort. Such a retreat makes TMS delivery difficult, if not impossible, in many cases.

While some TMS applications have employed robotic solutions, such solutions are employed merely to move the coil to a fixed location. The coil is held in this fixed location during TMS delivery and moved between applications of TMS. Such solutions rely on either immobilizing the head or maintaining a pressure against the head as discussed above.

### SUMMARY OF THE INVENTION

By employing a robotic arm and tracking system, a TMS solution according to the present invention allows for delivery of transcranial magnetic stimulation without pressing the TMS stimulation device against the head. Such contactless delivery is especially beneficial in certain clinical situations, such as depression or chronic pain, where the individual may experience discomfort or be averse to a device touching their head.

Further, TMS solutions according to embodiments of the present invention avoid problems associated with systems which require a pressure against the head to function. Within systems requiring pressure against the head, through error or otherwise, it may be determined that the TMS stimulation device should be against the head with too much force. This can lead to a situation where the device is pressed against the patient's head rather hard and the patient recoils. Such a recoil leads to TMS being stopped and the TMS stimulation device needing to be relocated until contact can be re-established only for the patient to recoil again which can lead to the patient being in an uncomfortable and awkward position.

In contrast, embodiments of the present invention provide for systems in which the TMS stimulation device essentially hovers above the head at a fixed location relative to the head. The head is tracked and that tracking is used to maintain the fixed hover location relative to the head. This hover provides for less discomfort and disturbance to a patient during TMS. For example, embodiments of the present invention provide for a substantial reduction in sound, vibration and heat transfer as there is essentially no contact with the head.

The invention is defined by the features of the independent claims. Some specific embodiments are defined in the dependent claims.

According to a first aspect of the present invention, there is provided an apparatus for facilitating delivery of Transcranial Magnetic Stimulation, TMS, comprising: a controller configured to be functionally connected to a tracking system and a multi-axis robotic arm having a TMS stimulation device affixed thereto; the controller being configured to:
- receive anatomical data for a person, said anatomical data defining a shape of at least the cranium of the person and an outer surface of the skin surrounding the cranium;
- receive information from the tracking system regarding a real-time position and orientation of the head of the person; and
- control the multi-axis robotic arm to maintain the TMS stimulation device at an optimal position and orientation relative to the head based on the anatomical data and the information regarding the real-time position and orientation of the head in order to target a predetermined location within the cranium with the TMS stimulation device;
wherein the optimal position and orientation is such that a surface of the TMS stimulation device is a predetermined distance from the outer surface of the skin.

According to a second aspect of the present invention, there is provided a method for facilitating delivery of Transcranial Magnetic Stimulation (TMS) via an apparatus comprising a controller, a tracking system and a multi-axis robotic arm having an affixed TMS stimulation device, the method comprising the steps of:
- receiving anatomical data for a person, said anatomical data defining a shape of at least the cranium of the person and an outer surface of the skin surrounding the cranium;
- receiving information from the tracking system regarding a real-time position and orientation of the head of the person; and
- sending control signals to control the multi-axis robotic arm to maintain the TMS stimulation device at an optimal position and orientation relative to the head based on the anatomical data and the information regarding the real-time position and orientation of the head in order to target a predetermined location within the cranium with the TMS stimulation device;
wherein the optimal position and orientation is such that a surface of the TMS stimulation device is a predetermined distance from the outer surface of the skin.

According to a third aspect of the present invention, there is provided a non-transitory computer readable medium having stored thereon a set of computer readable instructions that, when executed by at least one processor, cause an apparatus configured to be functionally connected to a tracking system and a multi-axis robotic arm having an affixed TMS stimulation device to at least:
- receive anatomical data for a person, said anatomical data defining a shape of at least the cranium of the person and an outer surface of the skin surrounding the cranium;
- receive information from the tracking system regarding a real-time position and orientation of the head of the person; and
- send control signals to control the multi-axis robotic arm to maintain the TMS stimulation device at an optimal position and orientation relative to the head based on the anatomical data and the information regarding the real-time position and orientation of the head in order to target a predetermined location within the cranium with the TMS stimulation device;
wherein the optimal position and orientation is such that a surface of the TMS stimulation device is a predetermined distance from the outer surface of the skin.

According to a fourth aspect of the present invention there is provided an apparatus for facilitating delivery of Transcranial Magnetic Stimulation (TMS) comprising: means for receiving anatomical data for a person, said anatomical data defining a shape of at least the cranium of the person and an outer surface of the skin surrounding the cranium; means for receiving information regarding a real-time position and orientation of the head of the person; and means for controlling a multi-axis robotic arm having an affixed TMS stimulation device to maintain the TMS stimulation device at an optimal position and orientation relative to the head based on the anatomical data and the information regarding the real-time position and orientation of the head in order to target a predetermined location within the cranium with the TMS stimulation device; wherein the optimal position and orientation is such that a surface of the TMS stimulation device is a predetermined distance from the outer surface of the skin.

### BRIEF DESCRIPTION OF THE DRAWINGS

FIGURE 1 illustrates an example apparatus capable of supporting at least some embodiments of the present invention;
FIGURES 2A - 2C illustrates an embodiment of the present invention, detailing the location of a TMS stimulation device relative to a head;
FIGURE 3 an example apparatus capable of supporting at least some embodiments of the present invention;
FIGURE 4 is a flow graph of a method in accordance with at least some embodiments of the invention.

### EMBODIMENTS

### DEFINITIONS

Throughout the present application reference may be made to a patient. It should be understood that this term is used for convenience and references the target of transcranial magnetic stimulation as enabled by embodiments of the present invention.

Within this context a TMS stimulation device includes but is not limited to a coil, or electromagnetic coil. The TMS stimulation device may comprise a housing, coil, other electronic components and a handle.

The outer surface of the skin may reference, for example, an outer layer of the cutis or an outer surface thereof. The outer surface of the skin may also be assumed to be, in some embodiments a layer of the cutis to ease in modelling. Such edges may be derived from medical imaging.

As discussed herein a position may be, for example, a place, location, point, point in space, etc. Similarly, an orientation may be, for example, an alignment or direction.

Embodiments of the present invention provide for a system which facilitates a consistent and more comfortable delivery of TMS. By tracking the head and taking into account not only the cranium but also the anatomy surrounding the cranium, a robotic arm can maintain a position and orientation of a TMS stimulation device such that it hovers a set distance away from said anatomy. This hovering causes less discomfort in the patient and can even allow for movement of the head during a round of stimulation between TMS pulse delivery, another factor increasing patient comfort during delivery of TMS.

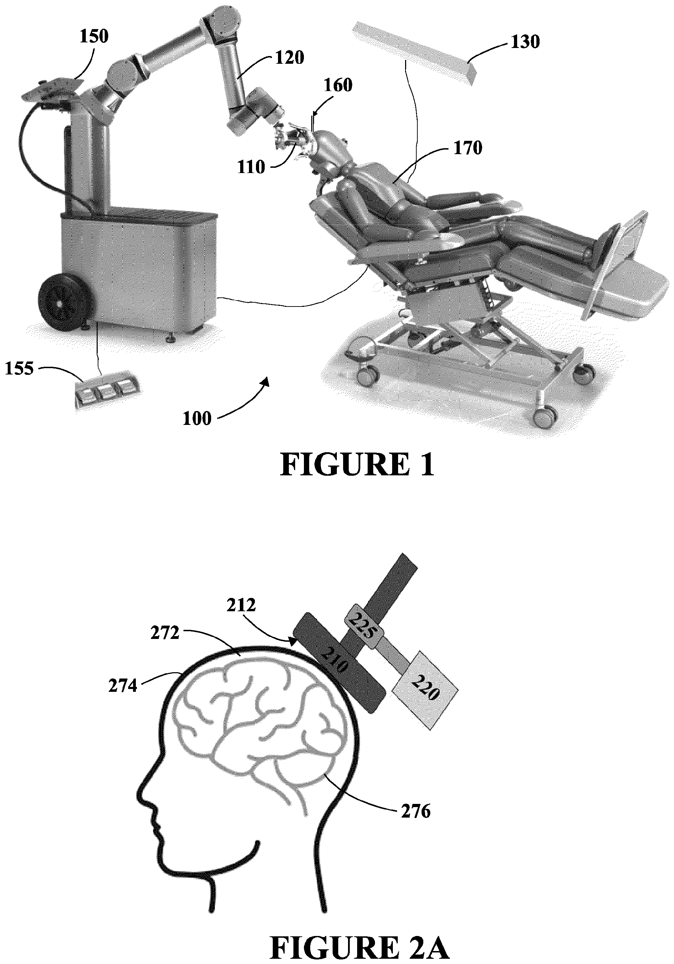

FIGURE 1 illustrates an apparatus 100 for facilitating delivery of Transcranial Magnetic Stimulation according to at least some embodiments of the present invention. As shown, the apparatus comprises: a controller 150 configured to be functionally connected to a tracking system 130 and a multi-axis robotic arm 120 having a TMS stimulation device 110 affixed thereto. The controller 150 is configured to receive anatomical data for a person 170, said anatomical data defining a shape of at least the cranium of the person and an outer surface of the skin surrounding the cranium. The controller is also configured to receive information from the tracking system 130 regarding a real-time position and orientation of the head of the person 170; and control the multi-axis robotic arm 120 to maintain the TMS stimulation device 110 at an optimal position and orientation relative to the head based on the anatomical data and the information regarding the real-time position and orientation of the head in order to target a predetermined location within the cranium with the TMS stimulation device 110. As seen in FIGURE 1, the optimal position and orientation is such that a surface of the TMS stimulation device 110 is a predetermined distance 160 from the outer surface of the skin.

In at least some embodiments of the present invention the anatomical data is derived from medical imaging, including but not limited to at least one of: magnetic resonance imaging MRI, computed tomography CT, X-Ray, and ultrasound. The medical imagery can have information which can be directly interpreted as representing the cranium and skin of an individual. In some instances, the anatomical data is derived by interpreting or adjusting medical imaging data. For example, the imaging data may have a clear indication of the cranium, but the skin may not be clearly shown, in which case assumptions can be made about the skin in order to provide for anatomical data concerning the skin surrounding the cranium. For example, an outer surface of the skin may be assumed to be a fixed distance from the cranium. Within certain embodiments the anatomical data defining a shape of at least the cranium of the person can be, for example, a shape of the brain or shape of the skull.

A wide variety of tracking systems may be employed with embodiments of the present invention. For example, a tracking system according to certain embodiments of the present invention is configured to track markers applied to the head of the individual. Such markers may be applied in a consistent location based on anatomical landmarks in order to provide for consistent mapping of anatomical data to the real-time position of the head.

Several methods and devices are known in which the location of a TMS stimulation device 110 can be determined and several are described in more detail at least in US 2008/0058582, "Transcranial magnetic stimulation induction coil device with attachment portion for receiving tracking device" which is herein incorporated by reference. At least some of these methods include tracking markers on or attached to the stimulating device 110. However, in at least some embodiments, tracking of the stimulating device is not necessary as a geometry and position of the robotic arm having the stimulation device can provide for a current location and orientation of the stimulating device. Additionally, markers can be attached to one or more locations on a subject's head. For example, a controller according to at least some embodiments of the present invention can derive or calculate, based on the known geometry and position of the robotic arm, the current location and orientation of the stimulating device. Such a calculation may be performed utilizing, for example, forward kinematics or inverse kinematics.

At least some embodiments of the present invention employ image recognition in order to track at least one of a subject's head and the TMS stimulation device. For example, a camera may be employed to capture images of the TMS stimulation device and/or subject's head and then image recognition techniques can derive a particular position and orientation for the TMS stimulation device and/or subject's head. Certain embodiments employ Artificial Intelligence (AI) or machine learning techniques to better track objects via image recognition. Some embodiments may even use image recognition in order to co-register the real-time information regarding the position and orientation of the patient's head with the anatomical data.

Anatomical data according to certain embodiments of the present invention may be adjusted. Such adjustment can take place either before receipt in a controller or after receipt. For example, controllers according to some embodiments are configured to adjust at least one of: the anatomical data for the person 170 defining the outer surface of the skin, and the predetermined distance from the outer surface of the skin, based on a current location of the surface of the TMS stimulation device. For example, the anatomical data can be updated or adjusted such that the outer surface of the skin is now considered to be at a point on a surface of the TMS stimulation device based on information regarding the location of the TMS stimulation device relative to the head of the individual.

Within some embodiments of the present invention the anatomical data is adjusted upon receipt of an update signal. Such a signal could be sourced, for example, from a manual switch, software switch, a sensor mounted on the face or surface of the TMS stimulation device, or a foot pedal 155 as illustrated in FIGURE 1. In embodiments employing a sensor, such as a touch sensor, on a face or surface of the TMS stimulation device, a calibration mode may be employed. In such a calibration mode the robotic arm may enter a "training mode" wherein movement is directed, either via control signals or by manually manipulating the robotic arm or TMS stimulation device. In this training mode the face or surface of the TMS stimulation device is brought into contact with the person's head at least one time, but potentially many times. At each contact a new data point is derived in order to update the anatomical data. A preferred method would be to bring the TMS stimulation device into contact with the patient's head at multiple different locations in order to update the anatomical data. Such a training mode may also be used to coordinate the anatomical data with the real-world tracking of the patient's head to, for example, account for errors in tracking or registration of anatomical data with real-world data. The use of training modes can also provide for boundary conditions and increases the safety and accuracy of the devices and methods described herein.

In certain embodiments the system is calibrated or anatomical data is adjusted by bringing the TMS stimulation device into contact with the patient's head at least once, for example at least three times. In some embodiments the TMS stimulation device is brought into contact with the patient's head at least 26 times. Such calibration or adjustment methods increase registration accuracy.

In some embodiments the controller 150 is further configured to perform at least one calibration step comprising sending control signals configured to move the TMS stimulation device 110 towards the head until a mechanical pressure or contact against the TMS stimulation device 110 is detected, for example, by at least one of: a sensor affixed to the TMS stimulation device and sensor(s) of the robotic arm.

FIGURE 2A shows a TMS stimulation device 210 in contact with the head of a person as may take place during calibration or anatomical data adjustment as discussed herein. A surface 212 of the TMS stimulation device is in contact with an outer surface of the skin 274 surrounding the cranium 272. As discussed, this surface 212 may comprise a sensor for detecting this contact. In some embodiments the sensor may be such that it provides information regarding the particular location on the surface 212 which is in contact with the outer surface of the skin 274.

As also illustrated in FIGURE 2A, the TMS stimulation device 210 is held by an adapter 225 which allows for connecting of the TMS stimulation device 210 to the robotic arm 220. Such an adapter 225 may be configured to interface with an end effector of the robotic arm 220. In certain embodiments the end effector of the robotic arm 220 may act as the adapter 225. This arrangement allows for positioning and orienting the TMS stimulation device 210 in a location so as to target a location within the cranium 272, for example a location within the brain 276, such as the cortex.

In addition, or as an alternative, to touch sensors affixed to the TMS stimulation device, sensors of the robotic arm itself are employed in certain embodiments. For example, sensors and/or motors of the robotic arm can determine when there is more than normal resistance to movement of the robotic arm, for example via monitoring one or more of the: voltage, current, acceleration, angular speed, angular acceleration of a motor and/or sensor. Such data obtained from the robotic arm can be used to trigger data update or provide other input. In certain embodiments of the present invention employing a touch sensor, the touch sensor does not need to provide a constant pressure sensing during TMS stimulation delivery due to the hover methods enabled by embodiments of the present invention. At least some embodiments employing a touch sensor will selectively disable the touch sensor during stimulation to avoid damage to the sensor itself or circuitry connected to the sensor.

In certain embodiments a user can bring the TMS stimulation device to a point where it touches a patient's head, for example manually as discussed above. At that point, the user, or even the patient, can provide a signal via switch, such as the foot pedal 155 of FIGURE 1. The user can either feel for the contact, ask the patient to indicate contact or one of the switches or inputs mentioned above may be employed for determining contact with the patient's head.

Adjustments to the anatomical data described herein may be used to account for errors in imaging or conversion of imaging. The adjustments may also provide accommodation for a patient's hair. For example, the system may indicate a certain hover distance is not possible, based on a sensor input saying that the TMS stimulation device is and/or will be in contact with something, for example, the patient or other entities in proximity to the patient. Alternatively, a user may notice that a certain hover distance, or predetermined distance from the outer surface of the skin indicated by the anatomical data, is not possible. Upon such an indication or determination, a user may independently or as prompted by the system, adjust the anatomical data. For example, as outlined in the adjustment and calibration techniques mentioned here.

Within certain embodiments the controller 150 is configured to perform a particular type of automated calibration. In such embodiments, the controller is configured to send control signals to move the TMS stimulation device 110 to a first location farther from the head than the optimal location and orientation, for example a location approximately 10 cm from the head. After this, the controller moves the TMS stimulation device 110 from the first location towards the head until a pressure against the TMS stimulation device 110 is detected. Within some embodiments the controller is configured such that the first movement happens at a first speed and the second movement happens at a second speed, slower than the first.

In at least some embodiments anatomical data defining the outer surface of the skin surrounding the cranium is derived via calibration steps. For example, in situations where the anatomical data is based on medical imaging, the skin layers in certain locations may not be well defined, or even not defined at all. As another example, a person's hair may cause the outer surface of the skin to be an unreasonable target for an offset as the hair is thick enough to cause pressure against the patient even when the TMS stimulation device is configured to hover above the outer surface of the skin.

In certain embodiments, the anatomical data defining the outer surface of the skin surrounding the cranium may initially be blank as received by the controller. For example, there may be no definitive data defining the outer surface of the skin. In such situations the controller can be configured to derive and/or update the data through the data adjustment methods described herein.

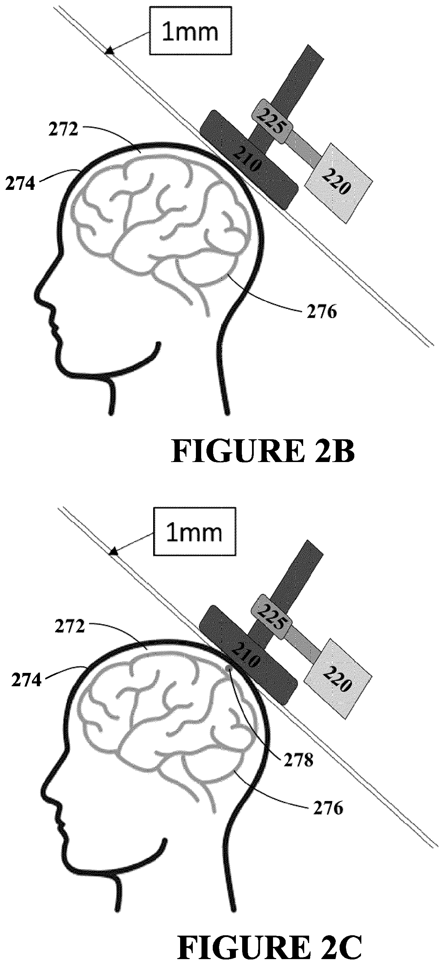

FIGURES 2B and 2C illustrate both the intended hover and situations when adjustment of data may be necessary. Within the figures, a cranium 272 is depicted, along with the skin 274 surrounding the cranium and the brain 276. Within figure 2B the TMS stimulation device 210 as held by the robotic arm 220 via adapter 225 at the predetermined distance of 1mm from the outer surface of the skin 274. The robotic arm maintains this position relative to the head throughout a session of TMS delivery. However, as shown in figure 2C, sometimes adjustment of anatomical data is necessary.

Within figure 2C the controller has received a target location 278, and thus an optimal position and orientation, which is actually in contact with the head or potentially even within the cranium 274. Even with the predetermined distance for offset of 1mm, when trying to position the TMS stimulation device 210 at the target location 278 resistance is encountered and detected due to contact with the head. This contact with the head may, in some embodiments trigger a warning, stop movement of the robotic arm and/or initiate a calibration or adjustment step as discussed herein.

At least some embodiments of the present invention employ a calibration step which uses anatomical data regarding the shape of the cranium as an initial limit to define anatomical data for the outer surface of the skin surrounding the cranium. For example, using calibration steps described herein, the TMS stimulation device may start at a set distance from the cranium and approach it until a pressure or contact is detected, thus defining the outer surface of the skin at that location. Certain embodiments may approach an initial optimal TMS delivery location and orientation until a contact is detected and then retreat a predetermined distance in order to arrive at the optimal position and orientation. Still other embodiments try to bring the coil to a reference location which would provide the strongest dose per energy delivered and then retreating a set distance once pressure is detected. The reference location may be, for example, a location directly on the cranium or skin surrounding the cranium. Such calibration steps serve to eliminate errors in imaging and tracking.

In certain embodiments of the present invention, the anatomical data defining the shape of the cranium is based on the anatomical data defining the outer surface of the skin surrounding the cranium. In other embodiments the anatomical data defining the outer surface of the skin surrounding the cranium is based on the anatomical data defining the shape of the cranium.

According to some embodiments of the present invention, the controller 150 is further configured to: receive the predetermined location within the cranium; and calculate the optimal position and orientation in which the surface of the TMS stimulation device 110 is the predetermined distance from the outer surface of the skin, based on the received predetermined location within the cranium. In certain embodiments the predetermined distance from the outer surface of the skin is 0.5 - 5 mm, preferably not more than 2 mm, most preferably not more than 1 mm.

At least some controllers according to embodiments of the present invention are configured to receive an intended dose of TMS to be delivered at the location within the cranium and calculate the necessary energy to be delivered to the TMS stimulation device such that the dose is delivered, adjusting for the predetermined distance from the outer surface of the skin. Certain embodiments are configured such that the controller sends signals configured to cause the TMS stimulation device to be energized and thus deliver TMS.

In addition to the benefits provided by the ability to hover during a TMS session, certain embodiments of the present invention provide for automated movement of the TMS stimulation device from one location to another via the robotic arm, for example from a location of a first stimulation dose delivery to a location of a second stimulation dose delivery. In such embodiments the controller 150 is configured to calculate at least one path for the multi-axis robotic arm 120 to move the TMS stimulation device 110 to the optimal location and orientation, wherein the path is calculated so that neither the multi-axis robot arm 120 nor the TMS stimulation device 110 contact the head or other parts of the patient. The controller 150 then sends control signals configured to move the multi-axis robotic arm 120 along the at least one path. Such automated movement may also be employed in certain embodiments which perform a calibration or anatomical data adjustment.

Certain embodiments of the present invention employ a camera, for example a 2/3D camera or other sensor, mounted to the robotic arm or TMS stimulation device which provides for collision detection and avoidance. Such a camera or sensor can detect or avoid collision with the patient or other objects within the environment, for example a chair the patient is sitting in. This detection and avoidance may also be incorporated into the calculation of paths for the robotic arm to move.

Some embodiments of the present invention provide for a controller 150 which is configured to define boundary conditions around at least one of: the head and TMS stimulation device 110; and prevent activation or energization of the TMS stimulation device 110 if the boundary conditions are violated. These boundary conditions may prevent situations wherein, through a lack of calibration or other error, the system is causing the TMS stimulation device 110 to be repeatedly pressed against the patient causing them to retreat from the device only for the system to repeatedly come into contact with the patient as they retreat. The boundary conditions may also provide for increased safety as the system may be configured to cease operation if, for example, a patient suddenly decided to leave. As an example, the boundary conditions may take the form of imaginary boxes or other defined volumes, potentially the size of matchboxes, which are drawn in space. In an initial condition these volumes or boxes are around a fixed point on the head or TMS stimulation device. If the fixed point leaves the box, the controller is configured to take further action. A controller may be configured, for example, to cause the robotic arm to retreat from the patient to prevent accidental collision in an instance where a boundary condition has been violated. The controller may also be configured to send a warning signal to an operator that a boundary condition has been violated, optionally ceasing other operations.

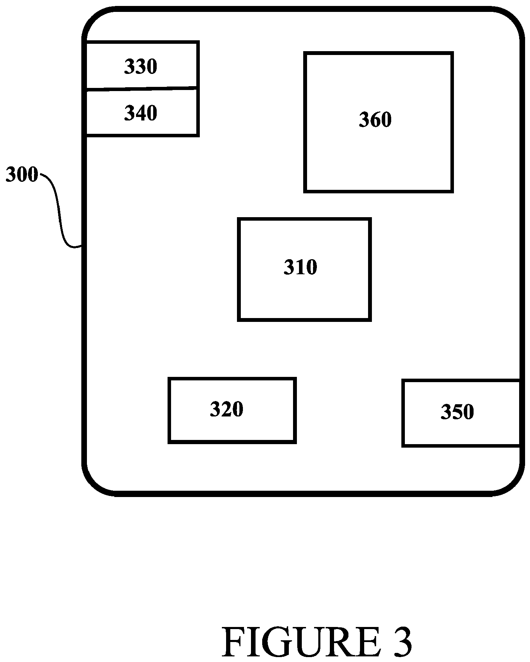

FIGURE 3 illustrates an example apparatus capable of supporting at least some embodiments of the present invention. Illustrated is device 300, which may comprise, for example, a computer device such as controller 150 of FIGURE 1. Comprised in device 300 is processor 310, which may comprise, for example, a single- or multi-core processor wherein a single-core processor comprises one processing core and a multi-core processor comprises more than one processing core. Processor 310 may comprise a Qualcomm Snapdragon 800 processor, for example. Processor 310 may comprise more than one processor. A processing core may comprise, for example, a Cortex-A8 processing core manufactured by Intel Corporation or a Brisbane processing core produced by Advanced Micro Devices Corporation. Processor 310 may comprise at least one application-specific integrated circuit, ASIC. Processor 310 may comprise at least one field-programmable gate array, FPGA. The aforementioned processor types are nonlimiting examples, alternatively an Intel i7 processor, or another suitable type of processor, may be employed.

Device 300 may comprise memory 320. Memory 320 may comprise randomaccess memory and/or permanent memory. Memory 320 may comprise at least one RAM chip. Memory 320 may comprise magnetic, optical and/or holographic memory. Memory 320 may be at least in part accessible to processor 310. Memory 320 may be means for storing information. Memory 320 may comprise computer instructions that processor 310 is configured to execute. When computer instructions configured to cause processor 310 to perform certain actions are stored in memory 320, and device 300 overall is configured to run under the direction of processor 310 using computer instructions from memory 320, processor 310 and/or its at least one processing core may be considered to be configured to perform said certain actions.

Device 300 may comprise a transmitter 330. Device 300 may comprise a receiver 340. Transmitter 330 and receiver 340 may be configured to transmit and receive, respectively, information in accordance with systems, for example transmitter 330 may transmit information to a monitor for display to a user, and/or receiver 340 may receive input information concerning a location and/or orientation of a further device.

Device 300 may comprise a near-field communication, NFC, transceiver 350. NFC transceiver 350 may support at least one NFC technology, such as NFC, Bluetooth, Wibree or similar technologies.

Device 300 may comprise user interface, UI, 360. UI 360 may comprise at least one of a display, a keyboard and a touchscreen. A user may be able to operate device 300 via UI 360, for example to start or terminate execution of programs.

Processor 310 may be furnished with a transmitter arranged to output information from processor 310, via electric leads internal to device 300, to other devices comprised in device 300. Such a transmitter may comprise a serial bus transmitter arranged to, for example, output information via at least one electric lead to memory 320 for storage therein. Alternatively, to a serial bus, the transmitter may comprise a parallel bus transmitter. Likewise, processor 310 may comprise a receiver arranged to receive information in processor 310, via electrical leads internal to device 300, from other devices comprised in device 300. Such a receiver may comprise a serial bus receiver arranged to, for example, receive information via at least one electric lead from receiver 340 for processing in processor 310. Alternatively, to a serial bus, the receiver may comprise a parallel bus receiver.

Device 300 may comprise further devices not illustrated in FIGURE 3. For example, where device 300 comprises a computer device, it may comprise at least one clock or auxiliary power unit, APU to provide battery power in case of mains power failure.

Processor 310, memory 320, transmitter 330, receiver 340, NFC transceiver 350 and/or UI 360 may be interconnected by electric leads internal to device 300 in a multitude of different ways. For example, each of the aforementioned devices may be separately connected to a master bus internal to device 300, to allow for the devices to exchange information. However, as the skilled person will appreciate, this is only one example and depending on the embodiment various ways of interconnecting at least two of the aforementioned devices may be selected without departing from the scope of the present invention.

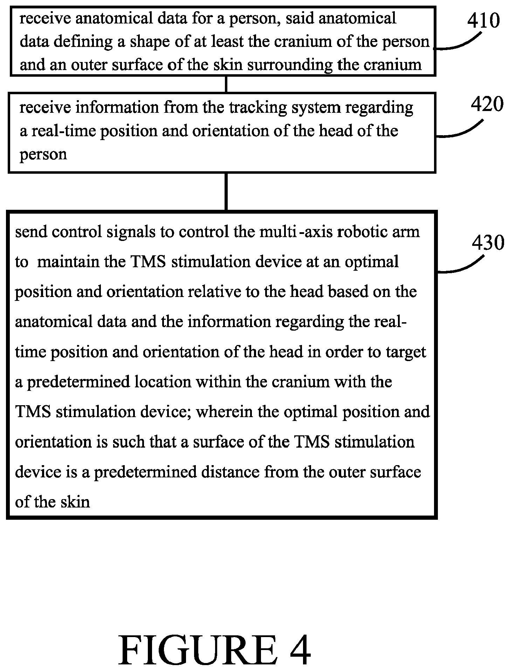

FIGURE 4 shows a flow graph of a method for facilitating delivery of Transcranial Magnetic Stimulation (TMS) via an apparatus 100 comprising a controller 150, a tracking system 130 and a multi-axis robotic arm 120 having an affixed TMS stimulation device 110, in accordance with at least some embodiments of the invention. The method is described with reference to figure 1, but it will be appreciated that it may be performed on another suitable apparatus. As shown in figure 4 the method comprises at least steps 410, 420 and 430. Step 410 involves receiving anatomical data for a person 170, said anatomical data defining a shape of at least the cranium of the person and an outer surface of the skin surrounding the cranium. Step 420 involves receiving information from the tracking system 130 regarding a real-time position and orientation of the head of the person 170. Step 430 involves sending control signals to control the multi-axis robotic arm 120 to maintain the TMS stimulation device 110 at an optimal position and orientation relative to the head based on the anatomical data and the information regarding the real-time position and orientation of the head in order to target a predetermined location within the cranium with the TMS stimulation device 110; wherein the optimal position and orientation is such that a surface of the TMS stimulation device (110) is a predetermined distance from the outer surface of the skin.

At least some methods according to the present invention further involve receiving the predetermined location within the cranium; and calculating the optimal position and orientation in which the surface of the TMS stimulation device (110) is the predetermined distance from the outer surface of the skin, based on the received predetermined location within the cranium.

Certain methods according to some embodiments of the present invention involve adjusting at least one of: the anatomical data for the person (170) defining the outer surface of the skin, and the predetermined distance from the outer surface of the skin, based on a current location of the surface of the TMS stimulation device. In some of these embodiments the adjusting is performed upon receipt of an update signal, such as a signal from a switch 155 or a pressure sensor affixed to the surface of the TMS stimulation device 110.

Some methods according to the present invention further comprise the step of performing at least one calibration step comprising sending control signals configured to move the TMS stimulation device 110 towards the head until a mechanical pressure or contact against the TMS stimulation device 110 is detected. Certain methods further comprise the steps of: receiving an intended dose of TMS to be delivered at the location within the cranium; calculating the necessary energy to be delivered to the TMS stimulation device 110 such that the dose is delivered, adjusting for the predetermined distance from the outer surface of the skin.

Some embodiments of the present invention comprise a non-transitory computer readable medium having stored thereon a set of computer readable instructions that, when executed by at least one processor, cause an apparatus configured to be functionally connected to a tracking system and a multi-axis robotic arm having an affixed TMS stimulation device to at least: receive anatomical data for a person, said anatomical data defining a shape of at least the cranium of the person and an outer surface of the skin surrounding the cranium; receive information from the tracking system regarding a real-time position and orientation of the head of the person; and send control signals to control the multi-axis robotic arm to maintain the TMS stimulation device at an optimal position and orientation relative to the head based on the anatomical data and the information regarding the real-time position and orientation of the head in order to target a predetermined location within the cranium with the TMS stimulation device; wherein the optimal position and orientation is such that a surface of the TMS stimulation device is a predetermined distance from the outer surface of the skin.

At least some embodiments of the present invention comprise a computer program configured to cause a method as described herein.

Within certain embodiments of the present invention a stimulation device is considered suitable for use as long as the curvature of the surface which faces the head during stimulation is not greater than an assumed or average curvature of a head. In other words, the stimulation device should not have a curve greater than the head of the individual to be stimulated to avoid problems maintaining a distance from the cranium of the individual.

Embodiments of the present invention find use with a wide variety of TMS stimulation devices employing a wide variety of coil designs. For example, butterfly coils, curved wing coils and figure 8 coil are well suited for use.

In at least some embodiments of the present invention, the controller comprises data regarding the dimensions of the TMS stimulation device as well as the robotic arm. The controller may also comprise data regarding the position and orientation of the TMS stimulation device relative to the robotic arm.

Within at least some embodiments of the present invention a target or target location is the location where the maximum e-field, electric field, is to be delivered by the TMS stimulation device when activated or energized. The target can be, for example, within the cortex. Within at least some embodiments the TMS stimulation device is positioned and oriented to target the predetermined location such that the maximum electric field of a predetermined stimulation will occur at that location. Within certain embodiments the dose is calculated such that a selected V/m dose is delivered at the target, for example the stimulation sight on the cortex. Some embodiments will take a dose originally intended for delivery with the TMS stimulation device being in contact with the head of the patient and compensate for the predetermined distance which the TMS stimulation device is maintained away from the head. Certain embodiments arrive at the predetermined distance by considering the intended dose and target location and determining a location and orientation offset from the patient's head which will deliver said dose.

At least some embodiments of the present invention provide for a system wherein an intended dose is provided and calculations are performed to determine a location touching the head of the patient which would provide the intended dose. The optimal location and orientation is then determined such that it is a predetermine distance from the head at the location touching the head. For example, 0.5 - 2 mm from the head.

At least some embodiments of the present invention provide for an apparatus for facilitating delivery of Transcranial Magnetic Stimulation (TMS) comprising:
- a multi-axis robotic arm;
- a TMS stimulation device affixed to the multi-axis robotic arm;
- a tracking system configured to track at least the head of an individual; and
- a controller functionally connected to the multi-axis robotic arm and the tracking system;
the controller being configured to:
- receive anatomical data for a person, said anatomical data defining a shape of at least the cranium of the person and an outer surface of the skin surrounding the cranium;
- receive from the tracking system information regarding a real-time position of the head of the person; and
- control the multi-axis robotic arm to target a predetermined location within the cranium with the TMS stimulation device based on the anatomical data and the information regarding the real-time position of the head in order to maintain the TMS stimulation device at an optimal position and orientation relative to the head;
wherein the optimal position and orientation is such that a surface of the TMS stimulation device is a predetermined distance from the outer surface of the skin.

Certain embodiments of the present invention provide for an apparatus for facilitating delivery of Transcranial Magnetic Stimulation (TMS) configured to be functionally connected to a tracking system and a multi-axis robotic arm having an affixed TMS stimulation device, the apparatus comprising at least one processing core, at least one memory including computer program code, the at least one memory and the computer program code being configured to, with the at least one processing core, cause the apparatus at least to:
- receive anatomical data for a person, said anatomical data defining a shape of at least the cranium of the person and cutis surrounding the cranium;
- receive information from the tracking system regarding a real-time position and orientation of the head of the person; and
- send control signals to control the multi-axis robotic arm to maintain the TMS stimulation device at an optimal position and orientation relative to the head based on the anatomical data and the information regarding the real-time position and orientation of the head in order to target a predetermined location within the cranium with the TMS stimulation device;
wherein the optimal position and orientation is such that a surface of the TMS stimulation device is a predetermined distance from the cutis

Suitable robotic devices for use within embodiments of the present invention comprise at least one motor, such as a servo motor, stepper motor, hydraulic motor, or pneumatic motor, linked to at least one joint, such as a rotary or linear joint. In a preferred embodiment, the robotic device is a robotic arm comprising at least five motors and at least five rotary joints which are connected by links arranged one after another. A separate or integrated controller, which controls the aforementioned motors, is used to control the robot movement. For example the robot UR5 by Universal robotics is a suitable robot for use with the invention. Other types of robotic manipulators are also usable with the invention.

As discussed herein, anatomical data for a person may be anatomical data of a person or derived for or of a person.

It is to be understood that the embodiments of the invention disclosed are not limited to the particular structures, process steps, or materials disclosed herein, but are extended to equivalents thereof as would be recognized by those ordinarily skilled in the relevant arts. It should also be understood that terminology employed herein is used for the purpose of describing particular embodiments only and is not intended to be limiting.

Reference throughout this specification to "one embodiment" or "an embodiment" means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment.

As used herein, a plurality of items, structural elements, compositional elements, and/or materials may be presented in a common list for convenience. However, these lists should be construed as though each member of the list is individually identified as a separate and unique member. Thus, no individual member of such list should be construed as a de facto equivalent of any other member of the same list solely based on their presentation in a common group without indications to the contrary. In addition, various embodiments and example of the present invention may be referred to herein along with alternatives for the various components thereof. It is understood that such embodiments, examples, and alternatives are not to be construed as de facto equivalents of one another, but are to be considered as separate and autonomous representations of the present invention.

Furthermore, the described features, structures, or characteristics may be combined in any suitable manner in one or more embodiments. In the following description, numerous specific details are provided, such as examples of lengths, widths, shapes, etc., to provide a thorough understanding of embodiments of the invention. One skilled in the relevant art will recognize, however, that the invention can be practiced without one or more of the specific details, or with other methods, components, materials, etc. In other instances, well-known structures, materials, or operations are not shown or described in detail to avoid obscuring aspects of the invention.

While the forgoing examples are illustrative of the principles of the present invention in one or more particular applications, it will be apparent to those of ordinary skill in the art that numerous modifications in form, usage and details of implementation can be made without the exercise of inventive faculty, and without departing from the principles and concepts of the invention. Accordingly, it is not intended that the invention be limited, except as by the claims set forth below.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", i.e. a singular form, throughout this document does not exclude a plurality.

### ACRONYMS LIST

- TMS: Transcranial Magnetic Stimulation

### REFERENCE SIGNS LIST

- 100: Apparatus
- 110: TMS Stimulation Device
- 120: Robotic Arm
- 130: Tracking System
- 150: Controller
- 155: Foot Pedal / Switch
- 160: Predetermined Distance
- 170: Person

- 210: TMS Stimulation Device
- 212: Surface of the TMS Stimulation Device
- 220: Robotic Arm
- 225: Adapter
- 272: Cranium
- 274: Outer Surface of the Skin
- 276: Brain
- 278: Target Location

- 300: Device
- 310: Processor
- 320: Memory
- 330: Transmitter
- 340: Receiver
- 350: Transceiver
- 360: UI

## Claims

1. An apparatus (100) for facilitating delivery of Transcranial Magnetic Stimulation, TMS, comprising:
- a controller (150) configured to be functionally connected to a tracking system (130) and a multi-axis robotic arm (120) having a TMS stimulation device (110) affixed thereto;
the controller (150) being configured to:
- receive anatomical data for a person (170), said anatomical data defining a shape of at least the cranium of the person and an outer surface of the skin surrounding the cranium;
- receive information from the tracking system (130) regarding a real-time position and orientation of the head of the person (170); and
- control the multi-axis robotic arm (120) to maintain the TMS stimulation device (110) at an optimal position and orientation relative to the head based on the anatomical data and the information regarding the real-time position and orientation of the head in order to target a predetermined location within the cranium with the TMS stimulation device (110);
**characterized in that** the optimal position and orientation is such that a surface of the TMS stimulation device (110) is a predetermined distance from the outer surface of the skin.

2. The apparatus of claim 1, wherein the controller (150) is further configured to:
- receive the predetermined location within the cranium; and
- calculate the optimal position and orientation in which the surface of the TMS stimulation device (110) is the predetermined distance from the outer surface of the skin, based on the received predetermined location within the cranium.

3. The apparatus of any preceding claim, wherein the predetermined distance from the outer surface of the skin is 0.5 - 5 mm, preferably not more than 2 mm, most preferably not more than 1 mm.

4. The apparatus of any preceding claim, wherein the controller (150) is further configured to adjust at least one of:
- the anatomical data for the person (170) defining the outer surface of the skin, and
- the predetermined distance from the outer surface of the skin,
based on a current location of the surface of the TMS stimulation device (110).

5. The apparatus of claim 4 wherein the controller (150) is further configured to perform the adjustment upon receipt of an update signal, such as a signal from a switch (155) or a pressure sensor affixed to the surface of the TMS stimulation device (110).

6. The apparatus of any preceding claim, wherein the controller (150) is further configured to perform at least one calibration step comprising sending control signals configured to move the TMS stimulation device (110) towards the head until a mechanical pressure or contact against the TMS stimulation device (110) is detected.

7. The apparatus of any preceding claim, wherein the controller (150) is further configured to:
- receive an intended dose of TMS to be delivered at the location within the cranium;
- calculate the necessary energy to be delivered to the TMS stimulation device (110) such that the dose is delivered, adjusting for the predetermined distance from the outer surface of the skin.

8. The apparatus of any preceding claim, wherein the controller (150) is further configured to:
- calculate at least one path for the multi-axis robotic arm (120) to move the TMS stimulation device (110) to the optimal location and orientation, wherein the path is calculated so that neither the multi-axis robot arm (120) nor the TMS stimulation device (110) contact the head; and
- send control signals configured to move the multi-axis robotic arm (120) along the at least one path.

9. The apparatus of any preceding claim, wherein the anatomical data is derived from medical imaging based on at least one of: magnetic resonance imaging MRI, computed tomography CT, X-Ray, and ultrasound.

10. A method for facilitating delivery of Transcranial Magnetic Stimulation, TMS, via an apparatus (100) comprising a controller (150), a tracking system (130) and a multi-axis robotic arm (120) having an affixed TMS stimulation device (110), the method comprising the steps of:
- receiving anatomical data for a person (170), said anatomical data defining a shape of at least the cranium of the person and an outer surface of the skin surrounding the cranium;
- receiving information from the tracking system (130) regarding a real-time position and orientation of the head of the person (170); and
- sending control signals to control the multi-axis robotic arm (120) to maintain the TMS stimulation device (110) at an optimal position and orientation relative to the head based on the anatomical data and the information regarding the real-time position and orientation of the head in order to target a predetermined location within the cranium with the TMS stimulation device (110);
**characterized in that** the optimal position and orientation is such that a surface of the TMS stimulation device (110) is a predetermined distance from the outer surface of the skin.

11. The method of claim 10, further comprising the steps of:
- receiving the predetermined location within the cranium; and
- calculating the optimal position and orientation in which the surface of the TMS stimulation device (110) is the predetermined distance from the outer surface of the skin, based on the received predetermined location within the cranium.

12. The method of claim 10 or 11, further comprising adjusting at least one of:
- the anatomical data for the person (170) defining the outer surface of the skin, and
- the predetermined distance from the outer surface of the skin,
based on a current location of the surface of the TMS stimulation device.

13. The method of claim 12 wherein the adjusting is performed upon receipt of an update signal, such as a signal from a switch (155) or a pressure sensor affixed to the surface of the TMS stimulation device (110).

14. The method of any of claims 10 - 13 further comprising the step of performing at least one calibration step comprising sending control signals configured to move the TMS stimulation device (110) towards the head until a mechanical pressure or contact against the TMS stimulation device (110) is detected.

15. The method of any of claims 10 - 14 further comprising the steps of:
- receiving an intended dose of TMS to be delivered at the location within the cranium;
- calculating the necessary energy to be delivered to the TMS stimulation device (110) such that the dose is delivered, adjusting for the predetermined distance from the outer surface of the skin.

16. A non-transitory computer readable medium having stored thereon a set of computer readable instructions that, when executed by at least one processor, cause an apparatus configured to be functionally connected to a tracking system and a multi-axis robotic arm having an affixed transcranial magnet stimulation, TMS, stimulation device to at least:
- receive anatomical data for a person, said anatomical data defining a shape of at least the cranium of the person and an outer surface of the skin surrounding the cranium;
- receive information from the tracking system regarding a real-time position and orientation of the head of the person; and
- send control signals to control the multi-axis robotic arm to maintain the TMS stimulation device at an optimal position and orientation relative to the head based on the anatomical data and the information regarding the real-time position and orientation of the head in order to target a predetermined location within the cranium with the TMS stimulation device;
**characterized in that** the optimal position and orientation is such that a surface of the TMS stimulation device is a predetermined distance from the outer surface of the skin.
